# EUROPÄISCHE PATENTSCHRIFT

(11) **EP 4 444 214 B1**
(45) Veröffentlichungstag und Bekanntmachung des Hinweises auf die Patenterteilung: **01.01.2025**
(21) Anmeldenummer: 24704712.9
(22) Anmeldetag: 07.02.2024
(51) Int. Cl.: A61B 50/20, A61B 50/33, A61B 50/34

(54) **INSTRUMENTENSIEB FÜR ÄRZTLICHE, INSBESONDERE CHIRURGISCHE INSTRUMENTE**
INSTRUMENT SIEVE FOR MEDICAL INSTRUMENTS, IN PARTICULAR SURGICAL INSTRUMENTS
TAMIS POUR INSTRUMENTS MÉDICAUX, EN PARTICULIER INSTRUMENTS CHIRURGICAUX

(30) Priorität: 14.02.2023 DE 102023103493
(43) Veröffentlichungstag der Anmeldung: 16.10.2024
(73) Patentinhaber: Aesculap AG, 78532 Tuttlingen (DE)
(72) Erfinder: WEISSER, Daniel, 78048 Villingen-Schwenningen (DE); STREIT, Eva, 78351 Bodman-Ludwigshafen (DE); KNITTEL, Timo, 78573 Wurmlingen (DE); DEUTSCHER, Hermann, 78579 Neuhausen ob Eck (DE)
(74) Vertreter: DREISS Patentanwälte PartG mbB
(86) Internationale Anmeldenummer: PCT/EP2024/053052
(87) Internationale Veröffentlichungsnummer: WO 2024/170380

(56) Entgegenhaltungen:
- WO-A1-2021/160274
- DE-A1- 102005 047 187
- DE-A1- 102007 030 863
- DE-A1- 102017 109 869
- US-A1- 2010 176 016

## Beschreibung

Die Erfindung betrifft ein Instrumentensieb für ärztliche, insbesondere chirurgische Instrumente und/oder Implantate zur Verwendung bei der Sterilgutversorgung im medizinischen Bereich, insbesondere OP-Bereich, mit einer Siebträgerstruktur mit einer Vielzahl von Sieböffnungen, mit einer den Instrumenten zugewandten Oberseite und einer von den Instrumenten abgewandten Unterseite der Siebträgerstruktur, mit wenigstens einer von der Oberseite der Siebträgerstruktur her an verschiedenen auswählbaren Stellen der Oberseite positionierbaren Vorrichtung zum Lagern und vorzugsweise zum Halten eines oder mehrerer Instrumente in einer bestimmungsgemäßen Anordnung auf dem Instrumentensieb, wobei die Vorrichtung ein Basisteil und ein daran lösbar befestigbares Instrumentenlagerteil umfasst, wobei das Basisteil an der Siebträgerstruktur lösbar festlegbar ist.

Derartige Instrumentensiebe, die häufig als Instrumentensiebkörbe oder Siebeinsätze für Sterilgutcontainer ausgebildet werden, spielen eine wichtige Rolle im sogenannten Sterilgutkreislauf ärztlicher und insbesondere chirurgischer Instrumente. Da sie die zu sterilisierenden Instrumente während der Sterilisation und auch danach im Zuge der Verbringung in das Sterilgutlager und von dort zum Ort der Verwendung, insbesondere einen Operationsraum, aufnehmen. Hierfür sind bereits sogenannte Organisationssysteme für Instrumentensiebe der vorstehend genannten Art vorgeschlagen worden, um die Instrumente in einer vorbestimmten Lage und Anordnung auf den Instrumentensieben vorzuhalten, bis sie unmittelbar vor ihrer Verwendung von medizinischem Personal entnommen werden. Vorbekannte Vorrichtungen zum Lagern eines oder mehrerer Instrumente auf dem Instrumentensieb sind entweder mittels Schraubverbindung an dem Instrumentensieb bzw. dessen Siebträgerstruktur befestigbar, was als aufwändig angesehen wird, oder sie werden von der Unterseite der Siebträgerstruktur her durch Sieböffnungen oder sonstige speziell ausgebildete Öffnungen durch die Siebträgerstruktur hindurchgesteckt, so dass sie auf der Oberseite des Instrumentensiebs nach oben vorstehen, um dort Instrumente lagern zu können. Beides impliziert eine Montage der Haltevorrichtung gewissermaßen von beiden Seiten des Instrumentensiebs. Die Haltevorrichtung oder die Haltevorrichtungen können demgemäß nur zu Beginn der Beladung mit Instrumenten auf der Siebträgerstruktur konfiguriert werden, da das Instrumentensieb hierfür verkippt und gewendet werden muss. Auch erweisen sich an der Unterseite des Instrumentensiebs angeordnete Montagemittel als störend; sie können insbesondere zu Abrieb, Beschädigung der Stellfläche oder bei ungleichmäßiger Verteilung zu einem Wackeln des Instrumentensiebs auf der Stellfläche führen.

Aus der US 2020/0030049A1 ist Träger für Sterilisationsgut bekannt, bei dem Pfosten mit Hakenabschnitten in Schlitze am Träger eingeclipst werden. An dem Pfosten können dann Teilelemente eingesetzt werden. Aus der DE 697 32 986 T2 ist ein Instrumentenhalter bekannt, bei dem ein Grundkörper aus Silikon und ein Edelstahl-Stützgerüst mit Rippen und Verriegelungszinken vorgesehen ist.

Weiter sind aus der DE 10 2005 047187 A1, US 2010/176016 A1, DE 10 2017 109869 A1, DE 10 2007 030863 A1 und der WO 2021/160274 A1 weitere Instrumentensiebe bzw. Sterilisationsgutträger bekannt.

Hiervon ausgehend liegt der vorliegenden Erfindung die Aufgabe zugrunde, ein Instrumentensieb der eingangs genannten Art im Hinblick auf die lösbare Anordnung der Vorrichtung zum Lagern eines oder mehrerer Instrumente bedienungsfreundlicher zu gestalten, wobei das lösbare Befestigen der Vorrichtung an der Siebträgerstruktur werkzeuglos ausgeführt werden soll.

Diese Aufgabe wird bei einem Instrumentensieb der genannten Art erfindungsgemäß dadurch gelöst, dass das Basisteil ein Blechbiegeteil ist und einen gegen die Oberseite der Siebträgerstruktur anlegbaren Grundschenkel und seitlich daran anschließend zwei einander gegenüberliegende Seitenschenkel aufweist, dass ausgehend von den Seitenschenkeln des Basisteils jeweils wenigstens eine Federzunge und vorzugsweise ein- oder beidseitig jeweils wenigstens zwei Federzungen ausgebildet sind, die sich ausgehend von den Seitenschenkeln des Basisteils in Richtung zu der Siebträgerstruktur hin erstrecken und gegenüber den Seitenschenkeln federnd auslenkbar sind, so dass das Basisteil mit seinem Grundschenkel auf der Oberseite der Siebträgerstruktur positionierbar ist, derart dass die wenigstens eine Federzunge in eine Sieböffnung hintergreifend eingreift, und dass dann lediglich durch Ausüben einer Druckkraft auf das Basisteil in Richtung auf die Oberseite der Siebträgerstruktur die gegenüberliegende Federzunge ausgelenkt wird und dabei selbsttätig in eine weitere Sieböffnung eindringt und dort mit einem die Sieböffnung begrenzenden Randbereich verrastet, so dass das Basisteil werkzeuglos und für den bestimmungsgemäßen Gebrauch unverlierbar an der Siebträgerstruktur befestigbar ist.

Es wird also erfindungsgemäß vorgeschlagen, das Basisteil der Haltevorrichtung mit nach unten, insbesondere schräg nach unten, in Richtung auf die Siebträgerstruktur erstreckten Federzungen auszubilden, mittels derer das Basisteil von der Oberseite der Siebträgerstruktur her mit der oder den Federzungen auf einer Seite des Basisteils in eine bzw. mehrere Sieböffnungen eingehakt werden kann und dann lediglich durch Druck auf das Basisteil in Richtung auf die Oberseite der Siebträgerstruktur die am gegenüberliegenden Seitenschenkel des Basisteils vorgesehenen Federzungen ausgelenkt und ebenfalls in eine Hintergriffstellung an der Siebträgerstruktur gebracht werden. Auf diese Weise ist eine werkzeuglose Anordnung und Montage eines jeweiligen Basisteils an dem Instrumentensieb denkbar. Im Anschluss hieran kann dann das Instrumentenlagerteil an dem Basisteil lösbar angeordnet werden, etwa in Richtung der Ebene der Siebträgerstruktur auf das Basisteil aufgeschoben werden oder aufgesteckt werden oder in sonstiger Weise kraftschlüssig oder formschlüssig an dem Basisteil angebracht werden. Es ist aber auch denkbar, dass das Basisteil mit bereits am Basisteil angeordnetem Instrumentenlagerteil in der vorstehend beschriebenen Weise auf dem Instrumentensieb lösbar festgelegt wird.

Im Hinblick auf eine wirtschaftliche Herstellbarkeit und Stabilität der Vorrichtung erweist es sich als vorteilhaft, wenn das Basisteil ein Blechbiegeteil ist und die Federzungen aus dem Blechbiegeteil ausgeklinkt sind. Solchenfalls lässt sich das Basisteil einschließlich seiner Federzungen aus einem metallischen Flachmaterialabschnitt durch Stanz- und Biegevorgänge ausbilden. Ein Biegefalz zwischen dem Grundschenkel und den beiden seitlich angrenzenden Seitenschenkeln verleiht dem Basisteil eine hohe Verwindungsstabilität, und zwar auch bei geringen Wandstärken des Basisteils von insbesondere 0,2 bis 1,0 mm, insbesondere von 0,25 bis 0,8 mm und insbesondere von 0,3 bis 0,5 mm. Weiter kann am oberen freien Ende der Seitenschenkel eine weitere Falzung, vorzugsweise nach innen, vorgesehen sein, was die Verwindungssteifigkeit weiter erhöht und zudem eine Schiebesitzaufnahme für das Instrumentenlagerteil begrenzen kann.

Weiter erweist es sich als vorteilhaft, wenn die Federzungen aus dem Seitenschenkel und aus dem Grundschenkel ausgeklinkt sind. Solchenfalls lässt sich die Federzunge hinreichend lang ausbilden, so dass sie in eine Sieböffnung des Instrumentensiebs eingreifen kann, ohne dass am freien Ende der Federzunge weitere Haltefortsätze und Hintergriffselemente gesondert vorgesehen oder angefügt werden müssen.

Um eine betriebssichere Festlegung der Vorrichtung bzw. ihres Basisteils an dem Instrumentensieb zu realisieren, erweist es sich als vorteilhaft, dass ein freies Ende vorzugsweise jeder der Federzungen zur Bildung eines hintergreifenden Bereichs oder einer hintergreifenden Sicke ausgebildet oder umgeformt ist. Somit vermag eine jeweilige Federzunge in eine formschlüssig wirkende Hintergriffstellung mit einem die betreffende Sieböffnung begrenzenden Randbereich der Siebträgerstruktur zu gelangen und unter der Wirkung einer rückfedernden Kraft der Federzunge darin auch betriebssicher zu verbleiben.

Es erweist sich weiter als vorteilhaft, wenn ein freies Ende vorzugsweise jeder der Federzungen um mehr als 90°, vorzugsweise um wenigstens 100°, vorzugsweise um wenigstens 110° zur Längserstreckung der Federzunge abgewinkelt ist und somit einen Hintergriff mit einem eine Sieböffnung begrenzenden Randbereich ausbildet.

Um das Festlegen des Basisteils an der Siebträgerstruktur einfach und betriebssicher und für die Bedienperson angenehm auszubilden, erweist es sich als vorteilhaft, wenn ein freies Ende vorzugsweise jeder der Federzungen eine Anlaufschräge der betreffenden Federzunge bildet, welche beim Aufdrücken des Basisteils in Richtung auf die Oberseite der Siebträgerstruktur gegen einen eine Sieböffnung begrenzenden Randbereich anläuft und dabei ausgelenkt wird, um in eine Hintergriffsstellung mit dem Randbereich zu rasten.

Wie eingangs bereits erwähnt, erweist es sich als zweckmäßig, wenn das Basisteil eine Rastaufnahme oder eine Schiebesitzaufnahme für das Instrumentenlagerteil bildet, wobei die Schiebesitzaufnahme seitlich durch die beiden Seitenschenkel des Basisteils und nach unten durch den Grundschenkel begrenzt ist. Weiter können einer oder vorzugsweise beide Seitenschenkel an ihrem oberen Randbereich einen Überstand nach innen, insbesondere durch Abfalzung des Seitenschenkels, aufweisen und hierdurch die Schiebesitzaufnahme nach oben hin begrenzen.

Bei dem Instrumentenlagerteil, welches an dem Basisteil lösbar befestigbar ist, handelt es sich nach einer Ausführungsform um ein polymeres Kunststoffteil, welches vorzugsweise mit oder aus Silikon ausgebildet ist. Das Instrumentenlagerteil ist zwar formstabil, es weist jedoch zweckmäßigerweise nachgiebig auslenkbare Haltebereiche für die Instrumente auf. Es ist zweckmäßigerweise auf zu lagernde Instrumente individuell angepasst und weist hierfür Haltestrukturen, insbesondere Haltearme und gegebenenfalls Ausnehmungen und Zentrierschrägen, zum Auflegen und insbesondere Einklipsen von Instrumenten auf.

Das Instrumentensieb kann eine prinzipiell beliebig ausgebildete Siebflächenstruktur aufweisen solange die Sieböffnungen periodisch angeordnet sind, so dass die Anordnung und Ausbildung, insbesondere der Abstand der Haltezungen voneinander, entsprechend dem Abstand der Sieböffnungen voneinander Rechnung ausgebildet werden können. Im Hinblick auf die Herstellung einer für Sterilisationszwecke weitgehend offenen Siebflächenstruktur erweist es sich als vorteilhaft, wenn das Instrumentensieb eine Siebträgerstruktur mit insbesondere rechteckförmigen und vorzugsweise quadratischen Sieböffnungen aufweist, die von gegenüber der Seitenlänge der Rechteckform oder gegenüber einer größten Abmessung der Sieböffnungen schmäleren Stegen begrenzt sind. Insbesondere beträgt eine Stegbreite der Stege höchstens 30%, insbesondere höchstens 20% einer größten Abmessung der Sieböffnungen, seien sie rechteckförmig oder nicht.

Es kann sich auch als vorteilhaft erweisen, wenn das Instrumentensieb in einer Draufsicht auf die Oberseite rechteckförmig ausgebildet ist mit einer dementsprechenden Längsrichtung und Querrichtung und wenn weiter die die Sieböffnungen begrenzenden Stege in einer Neigung von 45° zur Längsrichtung bzw. Querrichtung verlaufen. Solchenfalls können sich die Sieböffnungen ebenfalls mit einer Neigung von 45° zur Längsrichtung bzw. Querrichtung des Instrumentensiebs erstrecken. Wenn dann die Basisteile hingegen in der Längsrichtung oder in der Querrichtung ausgerichtet werden, so führt die rückfedernde Kraft der Federzungen gewissermaßen zu einer Selbstzentrierung der jeweiligen Federzunge in einem Eckbereich einer jeweiligen Sieböffnung.

Weiter kann es sich als vorteilhaft erweisen, wenn das Instrumentensieb eine 3-dimensional strukturierte, insbesondere gewellte Siebträgerstruktur umfasst. Solchenfalls ist das Basisteil im Bereich von Hochpunkten oder Bergen der Siebträgerstruktur anordenbar und befestigbar, wobei es nicht auf diesen Hochpunkten oder Bergen der Siebträgerstruktur ideal oben aufliegen muss, sondern es ist denkbar, dass die Siebträgerstruktur mit ihren Hochpunkten oder Bergen in Aussparungen des Grundschenkels des Basisteils eingreift.

In Weiterbildung dieses Gedankens erweist es sich als vorteilhaft, wenn in dem Basisteil Aussparungen ausgebildet sind, in welche die Siebträgerstruktur mit Erhebungen eingreifen kann, so dass hierdurch eine weitere Lagestabilisierung des Basisteils an der Siebträgerstruktur erzielbar ist.

Bei einer 3-dimensional strukturierten, insbesondere gewellten Siebträgerstruktur sind auch die in Sieböffnungen hintergreifend eingreifenden Federzungen bzw. deren hintergreifende Enden zwar auf der Unterseite, jedoch ungefähr im Bereich einer Erhebung der Siebträgerstruktur angeordnet und damit in einem Abstand zu einer Stellfläche des Instrumentensiebs. Hierdurch kann eine verschleißende Beanspruchung der Stellfläche durch die freien Enden der Federzungen sicher vermieden werden.

Das Instrumentensieb einschließlich dessen Siebträgerstruktur kann bei einer bevorzugten Ausführungsform aus einem metallischen Flachmaterial, insbesondere aus einem Metallblech, gebildet und insbesondere durch Schneid-, Stanz- und Biegevorgänge, insbesondere unter Anwendung von Laserschneidtechnik, hergestellt sein. Auf diese Weise kann eine Materialdicke der Siebträgerstruktur über deren Erstreckung, insbesondere über einen gesamten Bodenbereich eines Siebkorbs, konstant gehalten werden. Grundsätzlich sind aber auch geflochtene Siebträgerstrukturen denkbar und mitunter vorteilhaft.

Im Hinblick auf die vorstehend erwähnte Selbstzentrierung der Federzungen in Eckbereichen der Sieböffnungen, erweist es sich als vorteilhaft, wenn ein Abstand zweier benachbarter Federzungen eines Seitenschenkels einem Abstand zweier Kreuzungspunkte der die Sieböffnungen begrenzenden Stege entspricht oder das Doppelte oder ein ganzzahliges Vielfaches hiervon beträgt, so dass die Federzungen automatisch in jeweiligen Eckbereichen der Sieböffnungen positionierbar sind.

Weiter wird Schutz in Anspruch genommen für ein auf das Basisteil und dessen Federzungen abgestimmtes Löseinstrument als Bestandteil des Instrumentensiebs oder als Zubehörteil als solches, welches von innen durch Öffnungen in dem Basisteil hindurch gegen eine oder mehrere Federzungen im Bereich eines Seitenschenkels anlegbar ist und hierdurch diese Federzungen aus ihrer Hintergriffstellung auslenkbar sind, so dass das Basisteil aus einer Hintergriffsstellung bei dem Instrumentensieb gelöst werden kann.

Wie eingangs bereits angedeutet, kann das Instrumentensieb als Instrumentensiebkorb oder als ein Einsatz für einen Instrumentensiebkorb oder Instrumentenbehälter ausgebildet sein.

Als Teil eines Organisationssystems für ein Instrumentensieb der hier in Rede stehenden erfindungsgemäßen Art wird auch Schutz in Anspruch genommen für eine Vorrichtung als solche zum Lagern und vorzugsweise zum Halten eines oder mehrerer ärztlicher, insbesondere chirurgischer Instrumente in einer bestimmungsgemäßen Anordnung auf einem Instrumentensieb, wobei die Vorrichtung ein Basisteil und ein daran befestigbares Instrumentenlagerteil umfasst, wobei das Basisteil an dem Instrumentensieb lösbar festlegbar ist, wobei die Vorrichtung dadurch gekennzeichnet ist, dass das Basisteil ein Blechbiegeteil ist und einen gegen die Oberseite anlegbaren Grundschenkel und seitlich daran anschließend zwei einander gegenüberliegende Seitenschenkel aufweist, und dass ausgehend von den Seitenschenkeln des Basisteils jeweils wenigstens eine und vorzugsweise ein- oder beidseitig jeweils zwei Federzungen ausgebildet sind, die sich ausgehend von den Seitenschenkeln des Basisteils in Richtung auf die Siebträgerstruktur erstrecken und gegenüber den Seitenschenkeln federnd auslenkbar sind, so dass das Basisteil mit seinem Grundschenkel auf der Oberseite der Siebträgerstruktur positionierbar ist, derart dass die wenigstens eine Federzunge in eine Sieböffnung hintergreifend eingreift, und dass dann lediglich durch Ausüben einer Druckkraft auf das Basisteil in Richtung auf die Oberseite der Siebträgerstruktur die gegenüberliegende Federzunge ausgelenkt wird und dabei selbsttätig in eine weitere Sieböffnung eindringt und dort mit einem die Sieböffnung begrenzenden Randbereich verrastet, so dass das Basisteil werkzeuglos und für den bestimmungsgemäßen Gebrauch unverlierbar an der Siebträgerstruktur befestigbar ist.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung.

In der Zeichnung zeigt:
- Figur 1: eine perspektivische Darstellung eines erfindungsgemäßen Instrumentensiebs mit einer Siebträgerstruktur und mit einer Vorrichtung umfassend ein Basisteil und ein daran lösbar befestigbares Instrumentenlagerteil zum Lagern eines nicht dargestellten Instruments;
- Figur 2: eine perspektivische Darstellung des Basisteils der in Figur 1 dargestellten Vorrichtung;
- Figuren 3a-3c: verschiedene Darstellungen, welche die Montage des Halteteils an der Siebträgerstruktur des Instrumentensiebs verdeutlichen;
- Figuren 4a-4c: eine Seitenansicht, sowie Ansichten von oben und von unten auf das Instrumentensieb mit montiertem Basisteil;
- Figur 5: das Aufschieben des Instrumentenlagerteils auf das bereits auf der Siebträgerstruktur des Instrumentensiebs montierte Basisteil; und
- Figuren 6a, 6b: die Handhabung eines Löseinstruments zum Lösen des Basisteils von der Siebträgerstruktur des Instrumentensiebs.

Die Figuren zeigen ein Ausführungsbeispiel eines erfindungsgemäßen Instrumentensiebs 2, für ärztliche, insbesondere chirurgische, Instrumente, zur Verwendung bei der Sterilgutversorgung im medizinischen Bereich. Das Instrumentensieb 2 umfasst eine Siebträgerstruktur 4 mit einer Vielzahl von Sieböffnungen 6 und eine Vorrichtung 8 zum Lagern und vorzugsweise zum Halten eines in den Figuren nicht dargestellten Instruments während des Sterilgutkreislaufs. Die Vorrichtung 8 umfasst hierbei ein Basisteil 10 und ein daran lösbar befestigbares Instrumentenlagerteil 12. Das Basisteil 10 ist auf nachfolgend noch näher zu beschreibende Weise lösbar an der Siebträgerstruktur 4 befestigbar. Die Siebträgerstruktur 4 weist eine der Vorrichtung 8 und den nicht dargestellten Instrumenten zugewandte Oberseite 14 und eine hiervon abgewandte Unterseite 16 auf.

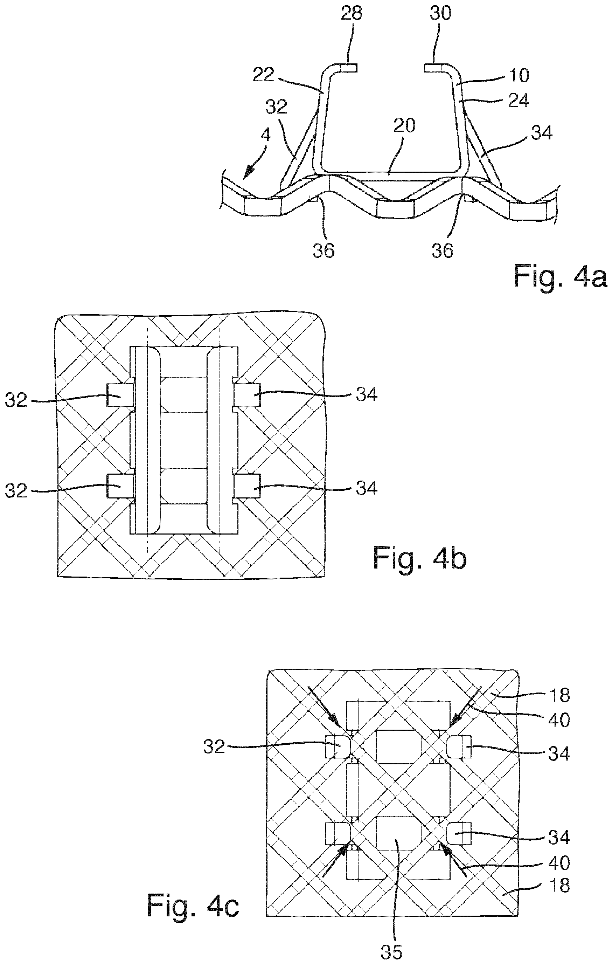

Im vorliegend dargestellten beispielhaften Fall ist die Siebträgerstruktur 4 gewellt ausgebildet und weist also eine dreidimensionale Wellenstruktur auf. Die Siebträgerstruktur 4 ist beispielhaft gebildet aus zwei Gruppen aus jeweils einer Vielzahl von parallel zueinander verlaufenden Stegen 18, wobei die Stege 18 der einen Gruppe rechtwinklig zu den Stegen 18 der anderen Gruppe verlaufen, so dass sie die Sieböffnungen 6 begrenzen. Ausgehend von einer zunächst ebenen Siebträgerstruktur 4 aus den Stegen 18 wurde dann die gewellte Struktur aufgeprägt. Trotz der dreidimensionalen Struktur können die Sieböffnungen 6 vorliegend beispielhaft als im Wesentlichen quadratisch bezeichnet werden (vgl. Figuren 4b, c), d.h. sie weisen bei senkrechter Projektion auf die Grundfläche der Siebträgerstruktur 4 eine quadratische Form auf.

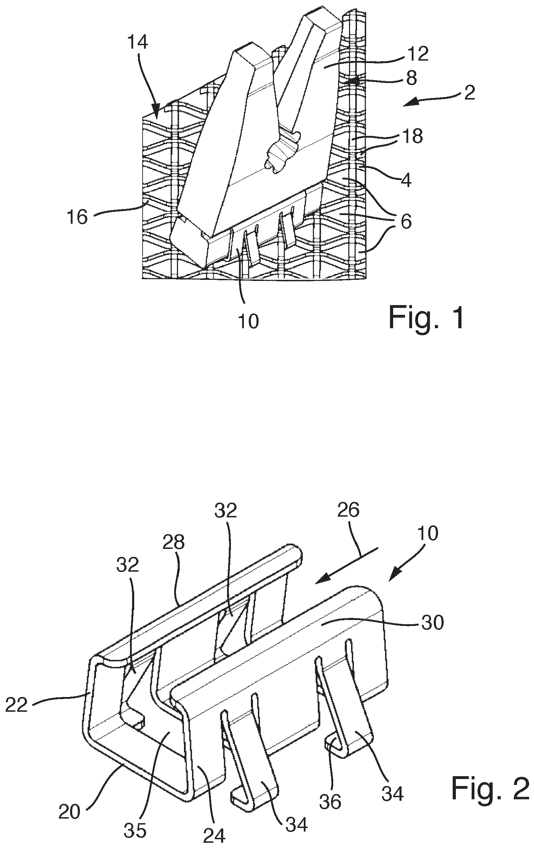

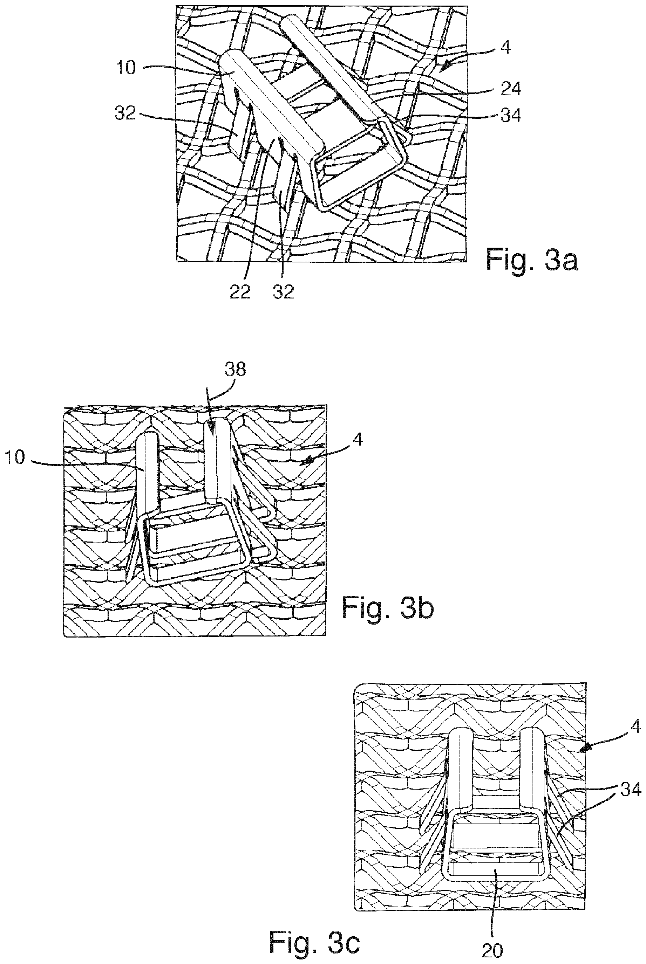

Figur 2, aber auch die weiteren Figuren, zeigen die Form und die Ausbildung des Basisteils 10. Es wurde im hier beispielhaft dargestellten Fall ausgehend von einem ebenen metallischen Flachmaterial gebildet, welches in Schneid-, Stanz- bzw. Biegevorgängen in die in Figur 2 dargestellte Form gebracht wurde. Das Basisteil 10 umfasst einen gegen die Oberseite 14 der Siebträgerstruktur 4 anlegbaren Grundschenkel 20 und seitlich daran anschließend zwei einander gegenüberliegende Seitenschenkel 22, 24, die im beispielhaft dargestellten Fall in Richtung eines Pfeils 26 betrachtet ungefähr eine U-Form begrenzen, die nach oben hin leicht verengt ist. Des Weiteren haben die Seitenschenkel 22, 24 an ihrem oberen freien Ende einen nach innen umgebogenen Randbereich 28 bzw. 30. Man erkennt des Weiteren, dass ausgehend von den Seitenschenkeln 22, 24 jeweils zwei Federzungen 32, 34 auf jeder Seite ausgebildet sind. Diese Federzungen 32, 34 sind aus dem Material des vormaligen Flachmaterialabschnitts durch einen Stanzschnitt ausgeklinkt und hinterlassen daher eine Aussparung 35 in dem Grundschenkel 20 und in den Seitenschenkeln 32, 34 des Basisteils 10. Sie sind nach außen gebogen, so dass sie sich ausgehend von einem oberen Bereich der Seitenschenkel 22, 24 in einem spitzen Winkel schräg nach unten in Richtung auf die Siebträgerstruktur 4 erstrecken und gegenüber den Seitenschenkeln 22, 24 federnd auslenkbar sind. Jede Federzunge 32, 34 ist an ihrem freien Ende nach innen umgebogen, und bildet einen hintergreifenden Bereich 36, mit dem eine jeweilige Federzunge 32, 34 in eine Sieböffnung 6 eingreifen und mit die Sieböffnung 6 begrenzenden Randbereichen, also mit den Stegen 18 der Siebträgerstruktur 4, eine formschlüssige Halteverbindung eingehen kann. Dies sei unter Verweis auf Figuren 3a bis 3c erläutert: Figur 3a zeigt die beispielhaft dreidimensional gewellte Siebträgerstruktur 4 und das Basisteil 10 mit seinen Federzungen 32, 34. Man erkennt in Figur 3a, dass das Basisteil 10 leicht gegen eine Ebene der Siebträgerstruktur 4 geneigt ist, so dass die in Figur 3a links dargestellten Federzungen 32 des Seitenschenkels 22 im Bereich eines Wellenbergs der Siebträgerstruktur 4 in zwei benachbarte Sieböffnungen 6 eingreifen und mit ihrem jeweiligen hintergreifenden Bereich 36 mit den Stegen 18 verhaken. Ausgehend von dieser Montagesituation braucht nun lediglich eine Druckkraft in Richtung eines in Figur 3b dargestellten Pfeils 38 auf das Basisteil 10 bzw. den anderen Seitenschenkel 24 in Richtung auf die Siebträgerstruktur 4 ausgeübt zu werden, damit die gegenüberliegenden Federzungen 34 mit ihren hintergreifenden Endbereichen 36 gegen die Stege 18 anlaufen und hierbei nach **außen ausgelenkt** werden und schließlich in die in Figur 3c dargestellte Hintergriffsituation gelangen, in der die Federzungen 34 nach Eingreifen in zwei Sieböffnungen wieder nach innen zurückfedern und hierdurch eine unverlierbare Montageposition an der Siebträgerstruktur 4 einnehmen. In dieser Montageposition ruht das Basisteil 10 gewissermaßen mit seinem Grundschenkel 20 im Bereich von Erhebungen der Siebträgerstruktur 4 und ist wieder parallel zur Ebene der Siebträgerstruktur 4 angeordnet. Das Basisteil ruht aber bei der beispielhaft dargestellten Ausführungsform nicht ganz oben auf den Hochpunkten der Erhebungen der Siebträgerstruktur 4, sondern auf den dorthin führenden Stegen ziemlich weit oben. Die Hochpunkte oder Gipfel der Erhebungen tauchen in Aussparungen 35 im Basisteil ein, die sich hier durch das Ausklinken der Federzungen 32 ergeben. Wenn das Basisteil länger erstreckt wäre und sich über mehrere Erhebungen der Siebträgerstruktur erstrecken würde, so könnten mehrere solcher Aussparungen in dem Basisteil ausgebildet sein, um ein solches Eintauchen oder Eingreifen der Hochpunkte oder Gipfel der Erhebungen zu ermöglichen.

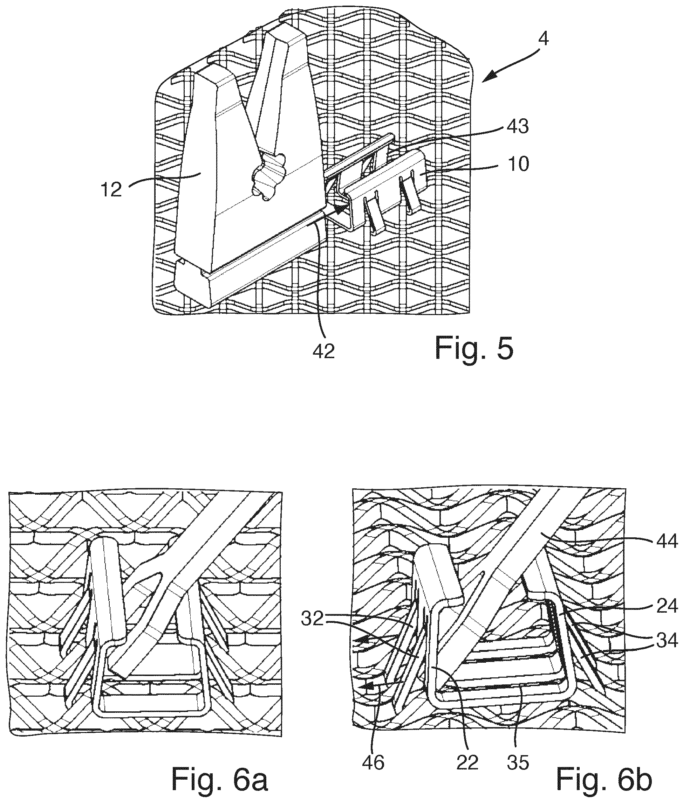

Sofern bei einer nicht dargestellten Ausführungsform die Federzungen nicht an zwei unmittelbar nebeneinanderliegenden Sieböffnungen des Siebkorbs angepasst sind, sondern zum Eingreifen in weiter voneinander beanstandete Sieböffnungen konzipiert sind, so kann das Basisteil mit einer oder mehreren zusätzlichen Aussparungen versehen werden, in die die Hochpunkte oder Gipfel der dazwischenliegenden Erhebungen einer 3-dimensional strukturierten Siebkörperstruktur eintauchen können. Dieses Eindringen verringert die Aufbauhöhe des Basisteils über dem Siebträgerstruktur und stabilisiert dessen Position gegenüber einer seitlichen Verschiebung (also in Richtung des Pfeils in Fig. 5), denn die Federzungen haben auch in Querrichtung eine gewisse Nachgiebigkeit, die in diese Richtung an sich nicht gewünscht ist.

Idealerweise liegen die Oberfläche der Gipfel der Erhebungen bei einer gewellten Siebträgerstruktur und die Oberfläche des Grundschenkels des Basisteils in einer Ebene; dies ermöglicht eine maximal reduzierte Aufbauhöhe des Basisteils und erlaubt noch das Einschieben des Instrumentenlagerteils, ohne dass dieses an den Gipfeln der Erhebungen anstößt. Für die Stabilität und Montagesicherheit des Instrumentenlagerteils ist es nämlich vorteilhaft, wenn dessen Unterseite auf der Oberseite des Grundschenkels des Basisteils auf- bzw. anliegt.

Die Figuren 4a bis 4c zeigen weitere Ansichten des Basisteils 10 im an der Siebträgerstruktur 4 montierten Zustand.

In Figur 4c verdeutlichen Pfeile 40, wie die Federzungen 32, 34 nach dem in Figur 3a, b dargestellten Eintauchen in die Sieböffnungen der dreidimensional gewellten Siebträgerstruktur 4 aufgrund ihrer rückfedernden Kraft entlang der Stege 18 selbstzentrierend in die dargestellte stabile Montageposition geführt werden. Dabei sind die freien Enden der Federzungen 32, 34 vorzugsweise gerundet bzw. gefast ausgebildet, da hierdurch die erforderliche Auslenkung der Federzungen 32, 34 beim Abgleiten an den relativ dazu schräg verlaufenden Stegen der gewellten Siebträgerstruktur verringert wird. Hierdurch kann man mit steiferen bzw. kräftigeren Federschenkeln arbeiten, und die Gefahr einer Beschädigung von Handschuhen oder Händen des Benutzers bei der Montage wird verringert, ohne dass dies einen wesentlichen Einfluss auf die Stabilität der Verankerung des Basisteils an der Siebträgerstruktur hat.

Figur 5 zeigt die Komplettierung der Haltevorrichtung 8, indem deren Instrumentenlagerteil 12 in Richtung des Pfeils 42 parallel zur Ebene der Siebträgerstruktur 4 auf das Basisteil 10 aufgeschoben werden kann. Das Basisteil 10 bildet dabei mit seinen oberen nach innen umgebogenen Randbereichen 28, 30 eine schwalbenschwanzartige Schiebesitzaufnahme für das hierzu komplementär ausgebildete Instrumentenlagerteil 12. Es sei aber ausdrücklich erwähnt, dass das Instrumentenlagerteil 12 und das Basisteil 10 auch in anderer Weise miteinander montiert und lösbar aneinander befestigt werden können. Insbesondere ist es auch denkbar, das Basisteil 10 mit bereits montiertem Instrumentenlagerteil 12 gegen die Siebträgerstruktur 4 in der beschriebenen Weise festzulegen.

Zum Lösen der Haltevorrichtung 8 bzw. des Basisteils 10 von der Siebträgerstruktur 4 ist es allerdings im beispielhaft dargestellten Fall zweckmäßig, das Instrumentenlagerteil 12 von dem noch montierten Basisteil 10 zu lösen, damit mittels eines in Figuren 6a, b dargestellten Löseinstruments 44 Zugriff auf die Federzungen 32, 34 genommen werden kann, um die Federzungen 32, 34 nach außen in Richtung der in Figur 6b angedeuteten Pfeile 46 nach außen auszulenken, so dass sie aus ihrer Hintergriffsituation mit den Stegen 18 der Siebträgerstruktur 4 freikommen und das Basisteil 10 von der Siebträgerstruktur 4 zunächst nach oben abgeschwenkt und dann entgegen der Pfeile 46 zur anderen Seite bewegt werden kann, damit auch die Federzungen 34 am gegenüberliegenden Seitenschenkel 24 aus ihrer Hintergriffsituation freikommen und das Basisteil 10 schließlich ganz abgenommen werden kann. Alternativ wäre es bei entsprechender Ausbildung der Länge und Vorspannung der Federzungen 32, 34 aber grundsätzlich auch denkbar, dass das Basisteil 10 werkzeuglos - mit oder ohne Instrumentenlagerteil 12 - in Richtung der Pfeile 46 nach links bewegt wird, so dass die in Figur 6b links dargestellten Federzungen 32 aus ihrer Hintergriffstellung mit den Stegen 18 freikommen und das Basisteil dann nach oben geschwenkt und zur gegenüberliegenden Seite hin von der Siebträgerstruktur 4 werkzeuglos abgenommen werden kann.

## Patentansprüche

1. Instrumentensieb (2) für ärztliche, insbesondere chirurgische Instrumente und/oder Implantate zur Verwendung bei der Sterilgutversorgung im medizinischen Bereich, insbesondere OP-Bereich,
mit einer Siebträgerstruktur (4) mit einer Vielzahl von Sieböffnungen (6),
mit einer den Instrumenten zugewandten Oberseite (14) und einer von den Instrumenten abgewandten Unterseite (16) der Siebträgerstruktur (4),
mit wenigstens einer von der Oberseite (14) der Siebträgerstruktur (4) her an verschiedenen auswählbaren Stellen der Oberseite (14) positionierbaren Vorrichtung (8) zum Lagern und vorzugsweise zum Halten eines oder mehrerer Instrumente in einer bestimmungsgemäßen Anordnung auf dem Instrumentensieb (2),
wobei die Vorrichtung (8) ein Basisteil (10) und ein daran lösbar befestigbares Instrumentenlagerteil (12) umfasst,
wobei das Basisteil (10) an der Siebträgerstruktur (4) lösbar festlegbar ist,
wobei das Basisteil (10) ein Blechbiegeteil ist und einen gegen die Oberseite (14) der Siebträgerstruktur (4) anlegbaren Grundschenkel (20) aufweist
**dadurch gekennzeichnet,**
**dass** das Basisteil (10) seitlich an den Grundschenkel (20) anschließend zwei einander gegenüberliegende Seitenschenkel (22, 24) aufweist,
**dass** ausgehend von den Seitenschenkeln (22, 24) des Basisteils (10) jeweils wenigstens eine Federzunge (32, 34) und vorzugsweise ein- oder beidseitig jeweils wenigstens zwei Federzungen (32, 34) ausgebildet sind,
wobei die Federzungen (32, 34) aus dem Blechbiegeteil ausgeklinkt sind und sich ausgehend von den Seitenschenkeln (22, 24) des Basisteils (10) in Richtung zu der Siebträgerstruktur (4) hin erstrecken,
**dass** die Federzungen (32, 34) gegenüber den Seitenschenkeln (22, 24) federnd auslenkbar sind, so dass das Basisteil (10) mit seinem Grundschenkel (20) auf der Oberseite (14) der Siebträgerstruktur (4) positionierbar ist, derart dass die wenigstens eine Federzunge (32) in eine Sieböffnung (6) hintergreifend eingreift, und
**dass** dann lediglich durch Ausüben einer Druckkraft auf das Basisteil (10) in Richtung auf die Oberseite (14) der Siebträgerstruktur (4) die gegenüberliegende Federzunge (34) ausgelenkt wird und dabei selbsttätig in eine weitere Sieböffnung (6) eindringt und dort mit einem die Sieböffnung (6) begrenzenden Randbereich verrastet, so dass das Basisteil (10) werkzeuglos und für den bestimmungsgemäßen Gebrauch unverlierbar an der Siebträgerstruktur (4) befestigbar ist.

2. Instrumentensieb (2) nach Anspruch 1, **dadurch gekennzeichnet, dass** die Federzungen (32, 34) gegenüber den Seitenschenkeln (22, 24) derart federnd auslenkbar sind, dass durch Ausüben der Druckkraft auf das Basisteil (10) in Richtung auf die Oberseite (14) der Siebträgerstruktur (4) die gegenüberliegenden Federzungen (34) mit ihren hintergreifenden Endbereichen (36) gegen die Stege (18) anlaufen und hierbei nach außen ausgelenkt werden und in eine Hintergriffsituation gelangen, in der die Federzungen (34) nach Eingreifen in Sieböffnungen (6) wieder nach innen zurückfedern.

3. Instrumentensieb (2) nach Anspruch 2, **dadurch gekennzeichnet, dass** die Federzungen (32, 34) aus dem Seitenschenkel (22, 24) und aus dem Grundschenkel (20) ausgeklinkt sind.

4. Instrumentensieb (2) nach einem oder mehreren der vorstehenden Ansprüche, **dadurch gekennzeichnet, dass** ein freies Ende vorzugsweise jeder der Federzungen (32, 34) zur Bildung eines hintergreifenden Bereichs (36) oder einer hintergreifenden Sicke ausgebildet oder umgeformt ist.

5. Instrumentensieb (2) nach einem oder mehreren der vorstehenden Ansprüche, **dadurch gekennzeichnet, dass** ein freies Ende vorzugsweise jeder der Federzungen (32, 34) um mehr als 90°, vorzugsweise um wenigstens 100°, vorzugsweise um wenigstens 110° zur Längserstreckung der Federzunge abgewinkelt ist und somit einen Hintergriff mit einem eine Sieböffnung (6) begrenzenden Randbereich ausbildet.

6. Instrumentensieb (2) nach einem oder mehreren der vorstehenden Ansprüche, **dadurch gekennzeichnet, dass** ein freies Ende vorzugsweise jeder der Federzungen (32, 34) eine Anlaufschräge der betreffenden Federzunge bildet, welche beim Aufdrücken des Basisteils (10) in Richtung auf die Oberseite (14) der Siebträgerstruktur (4) gegen einen eine Sieböffnung (6) begrenzenden Randbereich anläuft und dabei ausgelenkt wird, um in eine Hintergriffsstellung mit dem Randbereich zu rasten.

7. Instrumentensieb (2) nach einem oder mehreren der vorstehenden Ansprüche, **dadurch gekennzeichnet, dass** die einander gegenüberliegenden Seitenschenkel (22, 24) des Basisteils (10) eine U-Form begrenzen, die nach oben hin leicht verengt ist.

8. Instrumentensieb (2) nach einem oder mehreren der vorstehenden Ansprüche, **dadurch gekennzeichnet, dass** das Basisteil (10) eine Rastaufnahme oder eine Schiebesitzaufnahme (43) für das Instrumentenlagerteil (12) bildet, wobei die Schiebesitzaufnahme (43) seitlich durch die beiden Seitenschenkel (22, 24) des Basisteils (10) und nach unten durch den Grundschenkel (20) begrenzt ist.

9. Instrumentensieb (2) nach einem oder mehreren der vorstehenden Ansprüche, **dadurch gekennzeichnet, dass** das Instrumentenlagerteil (12) ein polymeres Kunststoffteil ist, vorzugsweise mit oder aus Elastomer und weiter vorzugsweise mit oder aus Silikon, ist.

10. Instrumentensieb (2) nach einem oder mehreren der vorstehenden Ansprüche, **dadurch gekennzeichnet, dass** das Instrumentenlagerteil (12) zwar formstabil ist, jedoch nachgiebig auslenkbare Haltebereiche für die Instrumente aufweist.

11. Instrumentensieb (2) nach einem oder mehreren der vorstehenden Ansprüche, **dadurch gekennzeichnet, dass** das Instrumentensieb (2) eine Siebträgerstruktur (4) mit insbesondere rechteckförmigen und vorzugsweise quadratischen Sieböffnungen (6) aufweist, die von gegenüber der Seitenlänge der Rechteckform oder gegenüber einer größten Abmessung der Sieböffnungen schmäleren Stegen (18) begrenzt sind.

12. Instrumentensieb (2) nach Anspruch 11, **dadurch gekennzeichnet, dass** das Instrumentensieb (2) in einer Draufsicht auf die Oberseite rechteckförmig ausgebildet ist mit einer dementsprechenden Längsrichtung und Querrichtung und dass die die Sieböffnungen (6) begrenzenden Stege (18) in einer Neigung von 45° zur Längsrichtung bzw. Querrichtung verlaufen.

13. Instrumentensieb (2) nach einem oder mehreren der vorstehenden Ansprüche, **dadurch gekennzeichnet, dass** das Instrumentensieb (2) eine 3-dimensional strukturierte, insbesondere gewellte Siebträgerstruktur (4) umfasst, insbesondere dass das Basisteil (10) im Bereich von Hochpunkten oder Bergen der Siebträgerstruktur (4) anordenbar und befestigbar ist.

14. Instrumentensieb (2) nach Anspruch 11, 12 oder 13, **dadurch gekennzeichnet, dass** ein Abstand zweier benachbarter Federzungen (32, 34) eines Seitenschenkels (22, 24) einem Abstand zweier Kreuzungspunkte der die Sieböffnungen (6) begrenzenden Stege (18) entspricht oder das Doppelte oder ein ganzzahliges Vielfaches hiervon beträgt, so dass die Federzungen (32, 34) automatisch in jeweiligen Eckbereichen der Sieböffnungen (6) positionierbar sind.

15. Instrumentensieb (2) nach einem oder mehreren der vorstehenden Ansprüche, **gekennzeichnet durch** ein Löseinstrument (44), welches von innen durch Öffnungen in dem Basisteil (10) hindurch gegen eine oder mehrere Federzungen (32, 34) im Bereich eines Seitenschenkels (22, 24) anlegbar ist und hierdurch diese Federzungen (32, 34) aus ihrer Hintergriffstellung auslenkbar sind, so dass das Basisteil (10) aus einer Hintergriffsstellung bei dem Instrumentensieb (2) gelöst werden kann.

16. Instrumentensieb (2) nach einem oder mehreren der vorstehenden Ansprüche, **dadurch gekennzeichnet, dass** es als Instrumentensiebkorb oder als ein Einsatz für einen Instrumentensiebkorb oder Instrumentenbehälter ausgebildet ist.

17. Vorrichtung (8) zum Lagern und vorzugsweise zum Halten eines oder mehrerer ärztlicher, insbesondere chirurgischer Instrumente und/oder Implantate in einer bestimmungsgemäßen Anordnung auf einem Instrumentensieb (2), wobei die Vorrichtung (8) ein Basisteil (10) und ein daran befestigbares Instrumentenlagerteil (12) umfasst, wobei das Basisteil (10) an eine Siebträgerstruktur (4) lösbar festlegbar ist, wobei das Basisteil (10) ein Blechbiegeteil ist und einen gegen eine Oberseite (14) der Siebträgerstruktur (4) anlegbaren Grundschenkel (20) aufweist, **dadurch gekennzeichnet, dass** das Basisteil (10) seitlich an den Grundschenkel (20) anschließend zwei einander gegenüberliegende Seitenschenkel (22, 24) aufweist, und
dass ausgehend von den Seitenschenkeln (22, 24) des Basisteils (10) jeweils wenigstens eine und vorzugsweise ein- oder beidseitig jeweils zwei Federzungen (32, 34) ausgebildet sind,
wobei die Federzungen (32, 34) aus dem Blechbiegeteil ausgeklinkt sind und sich ausgehend von den Seitenschenkeln (22, 24) des Basisteils (10) in Richtung auf die Siebträgerstruktur (4) erstrecken,
dass die Federzungen (32, 34) gegenüber den Seitenschenkeln (22, 24) federnd auslenkbar sind, so dass das Basisteil (10) mit seinem Grundschenkel (20) auf der Oberseite (14) der Siebträgerstruktur (4) positionierbar ist, derart dass die wenigstens eine Federzunge (32, 34) in eine Sieböffnung (6) hintergreifend eingreift, und
dass dann lediglich durch Ausüben einer Druckkraft auf das Basisteil (10) in Richtung auf die Oberseite (14) der Siebträgerstruktur (4) die gegenüberliegende Federzunge (32, 34) ausgelenkt wird und dabei selbsttätig in eine weitere Sieböffnung (6) eindringt und dort mit einem die Sieböffnung (6) begrenzenden Randbereich verrastet, so dass das Basisteil (10) werkzeuglos und für den bestimmungsgemäßen Gebrauch unverlierbar an der Siebträgerstruktur (4) befestigbar ist.

18. Vorrichtung (8) zum Lagern und vorzugsweise zum Halten eines oder mehrerer ärztlicher, insbesondere chirurgischer Instrumente und/oder Implantate in einer bestimmungsgemäßen Anordnung auf einem Instrumentensieb (2), wobei die Vorrichtung (8) ein Basisteil (10) und ein daran befestigbares Instrumentenlagerteil (12) umfasst, wobei das Basisteil (10) daran angepasst ist, an einer Siebträgerstruktur (4) eines Instrumentensiebs lösbar festlegbar zu sein, wobei
das Basisteil (10) ein Blechbiegeteil ist und einen gegen eine Oberseite (14) einer solchen Siebträgerstruktur (14) anlegbaren Grundschenkel (20) aufweist
**dadurch gekennzeichnet, dass** das Basisteil (10) seitlich an den Grundschenkel (20) anschließend zwei einander gegenüberliegende Seitenschenkel (22, 24) aufweist, und dass ausgehend von den Seitenschenkeln (22, 24) des Basisteils (10) jeweils wenigstens eine und vorzugsweise ein- oder beidseitig jeweils zwei Federzungen (32, 34) ausgebildet sind,
wobei die Federzungen (32, 34) aus dem Blechbiegeteil ausgeklinkt sind und gegenüber den Seitenschenkeln (22, 24) federnd auslenkbar sind und sich ausgehend von den Seitenschenkeln (22, 24) des Basisteils (10) in Richtung zu der Siebkörperstruktur hin und über den Grundschenkel hinaus erstrecken.

19. Vorrichtung (8) nach Anspruch 17 oder 18, **dadurch gekennzeichnet, dass** die Federzungen (32) mit den Seitenschenkeln (22, 24) einen spitzen Winkel einschließen.

20. Vorrichtung (8) nach Anspruch 18 oder 19, **dadurch gekennzeichnet, dass** die Federzungen (32, 34) gegenüber den Seitenschenkeln (22, 24) federnd auslenkbar sind und an ihren freien Enden zu dem Basisteil (10) hin abgewinkelt sind, insbesondere gerundet und/oder gefast ausgebildet sind.

21. Vorrichtung (8) nach einem oder mehreren der Ansprüche 17-19,
**dadurch gekennzeichnet, dass** das Basisteil (10) zwischen jeweils zwei gegenüberliegenden Federzungen (32, 34) mindestens eine Aussparung (35) aufweist, die insbesondere durch Ausklinken der beiden Federzungen (32, 34) gebildet ist.

22. Vorrichtung (8) nach einem oder mehreren der Ansprüche 17-20,
**dadurch gekennzeichnet, dass** in dem Basisteil (10) Aussparungen (35) ausgebildet sind, in welche die Siebträgerstruktur (4) mit Erhebungen eingreifen kann, so dass hierdurch eine weitere Lagestabilisierung des Basisteils an der Siebträgerstruktur erzielbar ist.

## Claims

1. A perforated instrument holder (2) for medical instruments, in particular surgical instruments and/or implants for use in supplying sterile goods in the medical field, in particular operating theaters,
comprising a perforated holder support structure (4) having a plurality of perforated holder openings (6),
comprising a top side (14), which faces the instruments, and a bottom side (16), which faces away from the instruments, of the perforated holder support structure (4),
comprising at least one device (8) which can be positioned, starting from the top side (14) of the perforated holder support structure (4), at various selectable locations on the top side (14) for storing and preferably for holding one or more instruments in an intended arrangement on the perforated instrument holder (2),
the device (8) comprising a base part (10) and an instrument bearing part (12) which can be releasably fastened thereto,
the base part (10) being releasably securable to the perforated holder support structure (4),
the base part (10) being a bent sheet metal part and having a main limb (20) that can be placed against the top side (14) of the perforated holder support structure (4)
**characterized in that**
the base part (10) has two opposite side limbs (22, 24) laterally adjoining the main limb (20),
**in that** proceeding from each of the side limbs (22, 24) of the base part (10) at least one flexible tongue (32, 34) and preferably at least two flexible tongues (32, 34) are formed on one or both sides,
the flexible tongues (32, 34) being notched out of the bent sheet metal part and extending from the side limbs (22, 24) of the base part (10) in the direction of the perforated holder support structure (4),
**in that** the flexible tongues (32, 34) are resiliently deflectable relative to the side limbs (22, 24) such that the base part (10) can be positioned with its main limb (20) on the top side (14) of the perforated holder support structure (4) such that the at least one flexible tongue (32) engages behind a perforated holder opening (6), and
**in that** only by exerting a compressive force on the base part (10) in the direction of the top side (14) of the perforated holder support structure (4) is the opposite flexible tongue (34) then deflected and automatically penetrates into a further perforated holder opening (6) where it latches with an edge region delimiting the perforated holder opening (6) such that the base part (10) can be fastened to the perforated holder support structure (4) without the use of tools and in a captive manner for the intended use.

2. Perforated instrument holder (2) according to claim 1, **characterized in that** the flexible tongues (32, 34) can be resiliently deflected relative to the side limbs (22, 24) in such a way that by exerting the compressive force on the base part (10) in the direction of the top side (14) of the perforated holder support structure (4), the opposite flexible tongues (34) run against the connecting pieces (18) with their rear-engaging end regions (36) and are thereby deflected outward and reach a rear-engagement situation in which the flexible tongues (34) spring back inward after engaging in perforated holder openings (6).

3. Perforated instrument holder (2) according to claim 2, **characterized in that** the flexible tongues (32, 34) are notched out of the side limb (22, 24) and out of the main limb (20).

4. Perforated instrument holder (2) according to one or more of the preceding claims, **characterized in that** a free end of preferably each of the flexible tongues (32, 34) is designed or shaped to form a rear-engaging region (36) or a rear-engaging bead.

5. Perforated instrument holder (2) according to one or more of the preceding claims, **characterized in that** a free end of preferably each of the flexible tongues (32, 34) is angled by more than 90°, preferably by at least 100°, preferably by at least 110° with respect to the longitudinal extension of the flexible tongue and thus engages behind an edge region delimiting a perforated holder opening (6).

6. Perforated instrument holder (2) according to one or more of the preceding claims, **characterized in that** a free end of preferably each of the flexible tongues (32, 34) forms a run-on slope of the corresponding flexible tongue, which, when the base part (10) is pressed in the direction of the top side (14) of the perforated holder support structure (4), runs against an edge region delimiting a perforated holder opening (6) and is deflected in order to latch with the edge region into a rear-engagement position.

7. Perforated instrument holder (2) according to one or more of the preceding claims, **characterized in that** the opposite side limbs (22, 24) of the base part (10) delimit a U shape which is slightly narrowed toward the top.

8. Perforated instrument holder (2) according to one or more of the preceding claims, **characterized in that** the base part (10) forms a latching receptacle or a sliding seat receptacle (43) for the instrument bearing part (12), the sliding seat receptacle (43) being delimited laterally by the two side limbs (22, 24) of the base part (10) and at the bottom by the main limb (20).

9. Perforated instrument holder (2) according to one or more of the preceding claims, **characterized in that** the instrument bearing part (12) is a polymeric plastics part, preferably comprising or consisting of elastomer and more preferably comprising or consisting of silicone.

10. Perforated instrument holder (2) according to one or more of the preceding claims, **characterized in that** the instrument bearing part (12) is dimensionally stable, but has resiliently deflectable retaining regions for the instruments.

11. Perforated instrument holder (2) according to one or more of the preceding claims, **characterized in that** the perforated instrument holder (2) has a perforated holder support structure (4) having in particular rectangular and preferably square perforated holder openings (6) which are delimited by connecting pieces (18) which are narrower than the side length of the rectangular shape or than the largest dimension of the perforated holder openings.

12. Perforated instrument holder (2) according to claim 11,
**characterized in that** the perforated instrument holder (2) is rectangular when the top side is viewed from above, with a corresponding longitudinal direction and transverse direction **and in that** the connecting pieces (18) delimiting the perforated holder openings (6) extend at an inclination of 45° to the longitudinal direction or transverse direction.

13. Perforated instrument holder (2) according to one or more of the preceding claims, **characterized in that** the perforated instrument holder (2) comprises a 3-dimensionally structured, in particular corrugated, perforated holder support structure (4), in particular **in that** the base part (10) can be arranged and fastened in the region of high points or peaks of the perforated holder support structure (4).

14. Perforated instrument holder (2) according to claim 11, claim 12 or claim 13, **characterized in that** a distance between two adjacent flexible tongues (32, 34) of a side limb (22, 24) corresponds to a distance between two intersection points of the connecting pieces (18) delimiting the perforated holder openings (6) or is twice or an integer multiple thereof, and therefore the flexible tongues (32, 34) can be automatically positioned in corresponding corner regions of the perforated holder openings (6).

15. Perforated instrument holder (2) according to one or more of the preceding claims, **characterized by** a release instrument (44) which can be placed from the inside via openings in the base part (10) against one or more flexible tongues (32, 34) in the region of a side limb (22, 24) and as a result these flexible tongues (32, 34) can be deflected from their rear-engagement position such that the base part (10) can be released from a position in which it engages behind the perforated instrument holder (2).

16. Perforated instrument holder (2) according to one or more of the preceding claims, **characterized in that** said holder is designed as a perforated instrument basket or as an insert for a perforated instrument basket or instrument container.

17. Device (8) for storing and preferably for holding one or more medical instruments, in particular surgical instruments and/or implants in an intended arrangement on a perforated instrument holder (2), the device (8) comprising a base part (10) and an instrument bearing part (12) that can be fastened thereto, the base part (10) being releasably securable to a perforated holder support structure (4), the base part (10) being a bent sheet metal part and having a main limb (20) that can be placed against a top side (14) of the perforated holder support structure **(4),characterized in that** the base part (10) has two opposite side limbs (22, 24) laterally adjoining the main limb (20), and
**in that** proceeding from each of the side limbs (22, 24) of the base part (10) at least one and preferably two flexible tongues (32, 34) are formed on one or both sides,
the flexible tongues (32, 34) being notched out of the bent sheet metal part and extending from the side limbs (22, 24) of the base part (10) in the direction of the perforated holder support structure (4),
**in that** the flexible tongues (32, 34) are resiliently deflectable relative to the side limbs (22, 24) such that the base part (10) can be positioned with its main limb (20) on the top side (14) of the perforated holder support structure (4) such that the at least one flexible tongue (32, 34) engages behind a perforated holder opening (6), and
**in that** only by exerting a compressive force on the base part (10) in the direction of the top side (14) of the perforated holder support structure (4) is the opposite flexible tongue (32, 34) then deflected and automatically penetrates into a further perforated holder opening (6) where it latches with an edge region delimiting the perforated holder opening (6) such that the base part (10) can be fastened to the perforated holder support structure (4) without the use of tools and in a captive manner for the intended use.

18. Device (8) for storing and preferably for holding one or more medical instruments, in particular surgical instruments and/or implants in an intended arrangement on a perforated instrument holder (2), the device (8) comprising a base part (10) and an instrument bearing part (12) that can be fastened thereto, the base part (10) being adapted to be releasably securable to a perforated holder support structure (4) of a perforated instrument holder,
the base part (10) being a bent sheet metal part and having a main limb (20) that can be placed against a top side (14) of such a perforated holder support structure (14), **characterized in that** the base part (10) has two opposite side limbs (22, 24) laterally adjoining the main limb (20), **and in that** proceeding from each of the side limbs (22, 24) of the base part (10) at least one and preferably two flexible tongues (32, 34) are formed on one or both sides,
the flexible tongues (32, 34) being notched out of the bent sheet metal part and being resiliently deflectable relative to the side limbs (22, 24) and extending from the side limbs (22, 24) of the base part (10) in the direction of the perforated holder body structure and beyond the main limb.

19. Device (8) according to claim 17 or claim 18, **characterized in that** the flexible tongues (32) form an acute angle together with the side limbs (22, 24).

20. Device (8) according to claim 18 or claim 19, **characterized in that** the flexible tongues (32, 34) can be resiliently deflected relative to the side limbs (22, 24) and are angled at their free ends toward the base part (10), in particular are rounded and/or chamfered.

21. Device (8) according to one or more of claims 17-19, **characterized in that** the base part (10) has at least one recess (35) between each two opposite flexible tongues (32, 34), which recess is formed in particular by notching the two flexible tongues (32, 34).

22. Device (8) according to one or more of claims 17-20, **characterized in that** recesses (35) are formed in the base part (10), in which the perforated holder support structure (4) can engage by means of raised portions such that the position of the base part on the perforated holder support structure can be further stabilized.

## Revendications

1. Tamis pour instruments (2) pour instruments médicaux, en particulier chirurgicaux et/ou implants, destiné à être utilisé pour la stérilisation dans le domaine médical, en particulier dans le domaine opératoire,
avec une structure de support de tamis (4) avec une pluralité d'ouvertures de tamis (6),
avec une face supérieure (14) tournée vers les instruments et une face inférieure (16) de la structure de support de tamis (4) opposée aux instruments,
avec au moins un dispositif (8) pouvant être positionné à partir de la face supérieure (14) de la structure de support de tamis (4) à différents endroits sélectionnables de la face supérieure (14) pour le stockage et de préférence pour le maintien d'un ou de plusieurs instruments dans une disposition conforme à l'usage prévu sur le tamis pour instruments (2),
dans lequel le dispositif (8) comprend une partie de base (10) et une partie de stockage d'instrument (12) pouvant être fixée de manière amovible à celle-ci,
dans lequel la partie de base (10) peut être fixée de manière amovible à la structure de support de tamis (4),
dans lequel la partie de base (10) est une partie pliée en tôle et présente une branche de base (20) pouvant être appliquée contre la face supérieure (14) de la structure de support de tamis (4)
**caractérisé en ce**
**que** la partie de base (10) présente deux branches latérales (22, 24) opposées l'une à l'autre se raccordant latéralement à la branche de base (20),
**que** respectivement au moins une languette élastique (32, 34) et de préférence d'un côté ou des deux côtés respectivement au moins deux languettes élastiques (32, 34) sont réalisées en partant des branches latérales (22, 24) de la partie de base (10),
dans lequel les languettes élastiques (32, 34) sont encliquetées à partir de la partie pliée en tôle et s'étendent à partir des branches latérales (22, 24) de la partie de base (10) en direction de la structure de support de tamis (4),
**que** les languettes élastiques (32, 34) peuvent être déviées élastiquement par rapport aux branches latérales (22, 24), de sorte que la partie de base (10) peut être positionnée avec sa branche de base (20) sur la face supérieure (14) de la structure de support de tamis (4), de telle sorte que la au moins une languette élastique (32) s'engage par l'arrière dans une ouverture de tamis (6), et
**qu'**ensuite, simplement en exerçant une force de pression sur la partie de base (10) en direction de la face supérieure (14) de la structure de support de tamis (4), la languette élastique (34) opposée est déviée et pénètre alors automatiquement dans une autre ouverture de tamis (6) et s'y enclenche avec une zone de bord délimitant l'ouverture de tamis (6), de sorte que la partie de base (10) peut être fixée sans outil et de manière imperdable sur la structure de support de tamis (4) pour l'utilisation conforme à l'usage prévu.

2. Tamis pour instruments (2) selon la revendication 1, **caractérisé en ce que** les languettes élastiques (32, 34) peuvent être déviées élastiquement par rapport aux branches latérales (22, 24), de telle sorte qu'en exerçant la force de pression sur la partie de base (10) en direction de la face supérieure (14) de la structure de support de tamis (4), les languettes élastiques (34) opposées viennent s'appliquer avec leurs zones d'extrémité (36) s'engageant par l'arrière contre les nervures (18) et sont ainsi déviées vers l'extérieur et arrivent dans une situation d'engagement par l'arrière, dans laquelle les languettes élastiques (34) reviennent élastiquement vers l'intérieur après s'être engagées dans les ouvertures de tamis (6).

3. Tamis pour instruments (2) selon la revendication 2, **caractérisé en ce que** les languettes élastiques (32, 34) sont encliquetées à partir de la branche latérale (22, 24) et de la branche de base (20).

4. Tamis pour instruments (2) selon l'une ou plusieurs des
revendications précédentes, **caractérisé en ce qu'**une extrémité libre de préférence de chacune des languettes élastiques (32, 34) est réalisée ou façonnée pour former une zone d'engagement par l'arrière (36) ou une moulure d'engagement par l'arrière.

5. Tamis pour instruments (2) selon l'une ou plusieurs des revendications précédentes, **caractérisé en ce qu'**une extrémité libre, de préférence de chacune des languettes élastiques (32, 34), est coudée à plus de 90°, de préférence à au moins 100°, de préférence à au moins 110° par rapport à l'extension longitudinale de la languette élastique et réalise ainsi un engagement par l'arrière avec une zone de bord délimitant une ouverture de tamis (6).

6. Tamis pour instruments (2) selon l'une ou plusieurs des revendications précédentes, **caractérisé en ce qu'**une extrémité libre de préférence de chacune des languettes élastiques (32, 34) forme un biseau d'attaque de la languette élastique concernée, lequel, lors de l'enfoncement de la partie de base (10) en direction de la face supérieure (14) de la structure de support de tamis (4), vient buter contre une zone de bord délimitant une ouverture de tamis (6) et est alors déviée pour s'encliqueter dans une position d'engagement par l'arrière avec la zone de bord.

7. Tamis pour instruments (2) selon une ou plusieurs des revendications précédentes, **caractérisé en ce que** les branches latérales (22, 24) opposées les unes aux autres de la partie de base (10) délimitent une forme en U qui est légèrement rétrécie vers le haut.

8. Tamis pour instruments (2) selon une ou plusieurs des revendications précédentes, **caractérisé en ce que** la partie de base (10) forme un logement d'encliquetage ou un logement d'assise coulissante (43) pour la partie de stockage d'instruments (12), dans lequel le logement d'assise coulissante (43) est limité latéralement par les deux branches latérales (22, 24) de la partie de base (10) et vers le bas par la branche de base (20).

9. Tamis pour instruments (2) selon une ou plusieurs des revendications précédentes, **caractérisé en ce que** la partie de stockage d'instruments (12) est une partie en plastique polymère, de préférence avec ou en élastomère et de préférence encore avec ou en silicone.

10. Tamis pour instruments (2) selon une ou plusieurs des revendications précédentes, **caractérisé en ce que** la partie de stockage d'instruments (12) est certes indéformable, mais présente des zones de maintien pour les instruments pouvant être déviées de manière flexible.

11. Tamis pour instruments (2) selon une ou plusieurs des revendications précédentes, **caractérisé en ce que** le tamis pour instruments (2) présente une structure de support de tamis (4) avec des ouvertures de tamis (6) notamment rectangulaires et de préférence carrées, qui sont délimitées par des nervures (18) plus étroites par rapport à la longueur latérale de la forme rectangulaire ou par rapport à une dimension maximale des ouvertures de tamis.

12. Tamis pour instruments (2) selon la revendication 11, **caractérisé en ce que** le tamis pour instruments (2), dans une vue de dessus de la face supérieure, est de forme rectangulaire avec une direction longitudinale et une direction transversale correspondantes, et que les nervures (18) délimitant les ouvertures detamis (6) s'étendent dans une inclinaison de 45° par rapport à la direction longitudinale ou à la direction transversale.

13. Tamis pour instruments (2) selon une ou plusieurs des revendications précédentes, **caractérisé en ce que** le tamis pour instruments (2) comprend une structure de support de tamis (4) structurée en 3 dimensions, en particulier ondulée, en particulier que la partie de base (10) peut être disposée et peut être fixée dans la zone des points hauts ou des berges de la structure de support de tamis (4).

14. Tamis pour instruments (2) selon la revendication 11, 12 ou 13, **caractérisé en ce qu'**une distance entre deux languettes élastiques (32, 34) voisines d'une branche latérale (22, 24) correspond à une distance entre deux points d'intersection des nervures (18) délimitant les ouvertures de tamis (6) ou est égale au double ou à un multiple entier de celle-ci, de sorte que les languettes élastiques (32, 34) peuvent être positionnées automatiquement dans des zones de coin respectives des ouvertures de tamis (6).

15. Tamis pour instruments (2) selon une ou plusieurs des revendications précédentes, **caractérisé par** un instrument de libération (44), lequel peut être appliqué de l'intérieur, à travers des ouvertures dans la partie de base (10), contre une ou plusieurs languettes élastiques (32, 34) dans la zone d'une branche latérale (22, 24) et, de ce fait, ces languettes élastiques (32, 34) peuvent être déviées de leur position d'engagement par l'arrière, de sorte que la partie de base (10) peut être libérée d'une position d'engagement par l'arrière dans le tamis pour instruments (2).

16. Tamis pour instruments (2) selon une ou plusieurs des revendications précédentes, **caractérisé en ce qu'**il est réalisé comme un panier formant tamis pour instruments ou comme un insert pour un panier formant tamis pour instruments ou un récipient à instruments.

17. Dispositif (8) pour le stockage et de préférence pour le maintien d'un ou de plusieurs instruments médicaux, en particulier chirurgicaux et/ou implants dans une disposition conforme à l'usage prévu sur un tamis pour instruments (2), dans lequel le dispositif (8) comprend une partie de base (10) et une partie de stockage d'instruments (12) pouvant être fixée à celle-ci, dans lequel la partie de base (10) peut être fixée de manière amovible à une structure de support de tamis (4), dans lequel la partie de base (10) est une partie pliée en tôle et présente une branche de base (20) pouvant être appliquée contre une face supérieure (14) de la structure de support de tamis (4), **caractérisé en ce que** la partie de base (10) présente deux branches latérales (22, 24) opposées l'une à l'autre, se raccordant latéralement à la branche de base (20), et
que respectivement au moins une et de préférence d'un côté ou des deux côtés respectivement deux languettes élastiques (32, 34) sont réalisées en partant des branches latérales (22, 24) de la partie de base (10),
dans lequel les languettes élastiques (32, 34) sont encliquetées à partir de la partie de tôle pliée et s'étendent à partir des branches latérales (22, 24) de la partie de base (10) en direction de la structure de support de tamis (4), que les languettes élastiques (32, 34) peuvent être déviées de manière élastique par rapport aux branches latérales (22, 24), de sorte que la partie de base (10) peut être positionnée avec sa branche de base (20) sur la face supérieure (14) de la structure de support de tamis (4), de telle sorte que la au moins une languette élastique (32, 34) s'engage par l'arrière dans une ouverture de tamis (6), et
qu'ensuite, simplement en exerçant une force de pression sur la partie de base (10) en direction de la face supérieure (14) de la structure de support de tamis (4), la languette élastique (32, 34) opposée est déviée et pénètre alors automatiquement dans une autre ouverture de tamis (6) et s'y enclenche avec une zone de bord délimitant l'ouverture de tamis (6), de sorte que la partie de base (10) peut être fixée sans outil et de manière imperdable sur la structure de support de tamis (4) pour l'utilisation conforme à l'usage prévu.

18. Dispositif (8) pour le stockage et de préférence pour le maintien d'un ou de plusieurs instruments médicaux, en particulier chirurgicaux, et/ou implants dans une disposition conforme à l'usage prévu sur un tamis pour instruments (2), dans lequel le dispositif (8) comprend une partie de base (10) et une partie de stockage d'instruments (12) pouvant être fixée à celle-ci, dans lequel la partie de base (10) est adaptée pour être fixée de manière amovible à une structure de support de tamis (4) d'un tamis pour instruments, dans lequel la partie de base (10) est une partie pliée en tôle et présente une branche de base (20) pouvant être appliquée contre une face supérieure (14) d'une telle structure de support de tamis (14)
**caractérisé en ce que** la partie de base (10) présente deux branches latérales (22, 24) opposées l'une à l'autre se raccordant latéralement à la branche de base (20), et que respectivement au moins une et de préférence d'un côté ou des deux côtés respectivement deux languettes élastiques (32, 34) sont réalisées à partir des branches latérales (22, 24) de la partie de base (10),
dans lequel les languettes élastiques (32, 34) sont encliquetées à partir de la partie pliée en tôle et peuvent être déviées élastiquement par rapport aux branches latérales (22, 24) et s'étendent en partant des branches latérales (22, 24) de la partie de base (10) en direction de la structure du corps de tamis et au-delà de la branche de base.

19. Dispositif (8) selon la revendication 17 ou 18, **caractérisé en ce que** les languettes élastiques (32) forment un angle aigu avec les branches latérales (22, 24).

20. Dispositif (8) selon la revendication 18 ou 19,
**caractérisé en ce que** les languettes élastiques (32, 34) peuvent être déviées élastiquement par rapport aux branches latérales (22, 24) et sont coudées à leurs extrémités libres en direction de la partie de base (10), en particulier sont réalisées de manière arrondie et/ou chanfreinée.

21. Dispositif (8) selon l'une ou plusieurs des revendications 17 à 19, **caractérisé en ce que** la partie de base (10) présente, entre respectivement deux languettes élastiques (32, 34) opposées, au moins un évidement (35) qui est notamment formé par encliquetage des deux languettes élastiques (32, 34).

22. Dispositif (8) selon une ou plusieurs des revendications 17 à 20, **caractérisé en ce que** des évidements (35) sont réalisés dans la partie de base (10), dans lesquels la structure de support de tamis (4) peut s'engager avec des bosses, de sorte qu'une stabilisation supplémentaire de la position de la partie de base sur la structure de support de tamis peut être obtenue.
